(19) Europäisches Patentamt
European Patent Office
Office européen des brevets

(11) **EP 4 157 138 B1**

(12) **EUROPEAN PATENT SPECIFICATION**

(45) Date of publication and mention
of the grant of the patent:
**29.04.2026 Bulletin 2026/18**

(21) Application number: **21740285.8**

(22) Date of filing: **26.05.2021**

(51) International Patent Classification (IPC):
*A61C 1/00* (2006.01)   *A61C 17/18* (2006.01)

(52) Cooperative Patent Classification (CPC):
**A61C 1/0084; A61C 17/18**

(86) International application number:
**PCT/US2021/034358**

(87) International publication number:
**WO 2021/242930 (02.12.2021 Gazette 2021/48)**

(54) **SYSTEM FOR IRRIGATION OF A TREATMENT AREA**

SYSTEM ZUR SPÜLUNG EINES BEHANDLUNGSBEREICHS

SYSTÈME D'IRRIGATION POUR LA ZONE DE TRAITEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.05.2020 US 202063030340 P**

(43) Date of publication of application:
**05.04.2023 Bulletin 2023/14**

(73) Proprietors:
• **Ivoclar Vivadent AG**
**9494 Schaan (LI)**
• **The Board of Trustees of the University
of Illinois
Urbana, IL 61801 (US)**

(72) Inventors:
• **KOEHLER, Thomas**
**78479 Reichenau (DE)**

• **SCHAEDLICH, Johannes**
**9016 St. Gallen (CH)**
• **BOCK, Thorsten**
**6800 Feldkirch (AT)**
• **YARIN, Alexander, L.**
**Clarendon Hills, IL 60514 (US)**
• **COOPER, Lyndon**
**Chicago, IL 60612 (US)**

(74) Representative: **Uexküll & Stolberg
Partnerschaft von
Patent- und Rechtsanwälten mbB
Beselerstraße 4
22607 Hamburg (DE)**

(56) References cited:
**US-A- 5 326 492       US-A- 5 326 492
US-A- 5 326 492       US-A- 5 876 744
US-A- 5 876 744       US-A1- 2004 138 079
US-A1- 2004 138 079    US-A1- 2006 254 988
US-A1- 2006 254 988    US-A1- 2006 254 988**

**Description**

**BACKGROUND OF THE DISCLOSURE**

Dental procedures or surgical procedures involving rotary, laser, kinetic, and ultrasonic instruments utilize water irrigation that becomes aerosolized during the procedure. Aerosols ($10^{-4}$ to 10 $\mu$m) are known to carry respiratory viruses (e.g., SARS, SARS-CoV-2). Aerosols and droplets generated can be carried for long distances during these procedures. Due to the current global health pandemic, and the potential for viruses or microorganisms in the future with a similar vector, the risk of transmission of disease during these dental/surgical procedures is receiving heightened attention. Specifically, during the performance of certain dental/surgical procedures, aerosolization can arise from the use of water in the procedures. With the potential of aerosol droplets carrying viruses or other microbes, extensive time and cost may need to be expended for infection management and as such, strategies are needed for safely carrying out dental and other surgical procedures, particularly involving patients carrying contagious microbes, such as SARS-CoV-2.

Furthermore, the generation of droplets and aerosols during handling of dental rotary, kinetic and ultrasonic equipment may reduce visibility of the working field for the operator, especially when using magnifying loupes. Aerosols and droplets also lead to misting of glasses, goggles and face shields and contaminate the patient's body and clothing and nearby working surfaces. Prior art is exemplified by US 2006/254988 A1.

**SUMMARY OF THE DISCLOSURE**

The invention is as defined in the appended claims. The present disclosure provides compositions and systems suitable for irrigation during dental or surgical procedures, where the treatment area is frequently irrigated with aqueous compositions. Also provided are methods of using the compositions. The compositions, systems and methods are particularly useful where there may be a potential risk of microbial transfer, such as viral or bacterial transfer.

The compositions of the present disclosure are aqueous compositions comprising, consisting essentially of, or consisting of one or more polymers. Polymers may be natural or synthetic. Data is provided herein to demonstrate that use of aqueous compositions of polymers reduces aerosolization.

In an aspect, this disclosure provides a system for irrigation of a treatment area. For example, the system may comprise waterlines for use in a dental unit configured to deliver an aqueous composition comprising, consisting essentially of or consisting of one or more polymers. The compositions may be released into the dental treatment area under pressures routinely used during dental procedures. In various embodiments, a system may comprise a dental unit water bottle (also referred to herein as reservoir) comprising aqueous compositions comprising polymers, and waterlines for delivery of the composition to the dental treatment area. The system may further comprise a control means, such as a dental handpiece, for controlling the release or the flow of the composition into the dental treatment area.

The composition and systems may be used in surgical/dental procedures for irrigation of treatment areas. For example, the compositions may be used where there is likelihood of generation of aerosols due to irrigation with water under rotary, ultrasonic or kinetic agitation. Reduction or elimination of aerosols at the point of generation will aid in preventing the airborne/aerosol transmission of viruses. Currently, during the COVID 19 pandemic, elective surgery or dentistry is frequently postponed. Alternatively, unusual and extensive protective measures are used which require permanent or mobile installation in the operatory, increasing cost and time needed for surgical or dental procedures. Using the methods, compositions, and systems described herein will aid in normalization of dental practice by reducing the risk of aerosol disease transmission to patients, health care workers and by-standers. In addition, the reduction of aerosols during dental treatment improves field of visibility for the operator, allowing more precise and timely operation. The reduced aerosol and droplet formation is also beneficial to the patient by preventing contamination of exposed body parts or clothing with airborne residues from dental procedures.

**BRIEF DESCRIPTION OF THE FIGURES**

For a fuller understanding of the nature and objects of the disclosure, reference should be made to the following detailed description taken in conjunction with the accompanying figures.

Figure 1 shows a schematic of a setup for testing of the compositions for their effect on aerosol reduction.
Figure 2 shows an image of aerosol formation in the presence of the indicated materials/compositions: water (on the left: full-scale aerosolization) and a very dilute PEO solution on the right (only big drops are formed, no fine aerosol).
Figure 3 shows an image of aerosol formation in the presence of the indicated materials/compositions. (Left) Practically complete, and complete (right) suppression of aerosol formation when PEO concentration is gradually increased.
Figure 4 shows the experimental set up used to investigate travel length of aerosols and droplets formed during ultrasonic agitation at a dental scaler tip. (Left) Glass slide used for drop collection, (middle) a frame in the sequence of recorded images of aerosol formation when water is used as an irrigation material, (right) schematic of the key element of the experimental setup.

Figure 5 shows efficiency of cleaning on a zirconia block model, varnished with dyed Fluorprotector S (upper part of zirconia not cleaned). Comparison of water against 0.1% of PEG 600 kDa solution.

Figure 6 shows a system according to an embodiment of the present disclosure, wherein the system includes an independent reservoir.

Figure 7 shows a system according to another embodiment of the present disclosure, and configured "in-line" with a dental unit waterline.

Figure 8 shows a system according to another embodiment of the present disclosure, and configured to be connected to introduce the aqueous composition into a dental unit waterline.

## DESCRIPTION OF THE DISCLOSURE

Although disclosed subject matter is described herein in terms of certain embodiments and examples, other embodiments and examples, including embodiments and examples that do not provide all of the benefits and features set forth herein, are also within the scope of this disclosure. Various structural, logical, and process step may be made without departing from the scope of the disclosure.

Every numerical range given throughout this specification includes its upper and lower values, as well as every narrower numerical range that falls within it, as if such narrower numerical ranges were all expressly written herein, and, unless described otherwise, every value is included to the tenth of the value of the lower limit.

In accordance with the principles herein, the present disclosure provides systems for irrigation of a treatment area. The systems deliver suitable compositions, *i.e.,* aqueous solutions and mixtures thereof (synthetic polymers) in suitable concentrations, which are provided herein, that can reduce or eliminate the number of aerosolized droplets produced during dental/surgical irrigation procedures. The systems provide increased safety of dental/surgical irrigation procedures. Reduction or elimination of aerosols can reduce and/or prevent the airborne/aerosol transmission of viruses. While not intending to be bound by any particular theory, it is considered that the increased elongational viscosity of dental/surgical irrigation solutions may contribute to a reduction or elimination of aerosolized droplets during dental/-surgical irrigation procedures. More precisely, aerosolization may be suppressed or eliminated by the manipulation of an irrigation medium's elongational viscosity (i.e. its submission to extensional stress, as is present during droplet formation from liquids) by addition of high molecular weight substances (e.g., polymers).

Dentistry uses rotary, ultrasonic, kinetic, and laser-based instruments in the daily, routine treatment of patients. All of these require water irrigation or the use of air/water syringes to cool the tooth surface and/or to wash away debris from the tooth. In providing water irrigation, abundant and continuous aerosols are generated from the high pressure differences involved or by the high velocities of moving parts. These aerosols may extend for many feet beyond the patient's mouth and the area where the dentist is working. Multiple viruses, including the SARS-CoV-2 virus, and the related COVID-19 pandemic have spread throughout the United States and the rest of the world. Several investigations have demonstrated the SARS-CoV-2 virus is spread by both droplet (visible drops) and aerosol transmission. The generation of aerosols in dentistry-an unavoidable part of most dental/surgical facial cosmetic and irrigation treatments-creates a high-risk situation by potentially spreading the virus long distances to other people.

The United States Centers for Disease Control and Prevention (CDC) and the Occupational Safety and Health Administration (OSHA) consider dental procedures to be of "highest risk" in the potential spreading of SARS-CoV-2 and other respiratory viruses. There are several ways to reduce or eliminate the virus; 1) cease or postpone dentistry (public and personal health risk), 2) screen patients immediately prior to dental treatment (but appropriate testing is not yet available), 3) block or remove the virus containing aerosol by engineering controls together with stringent PPE use.. As described herein, by altering the physical response of water to the rotary or ultrasonic forces that are used in dentistry, the generation of aerosol droplets and the distance any aerosol may spread beyond the point of generation may be suppressed or completely eliminated.

Compared to other administrative and engineering methods, the advantages of the compositions herein include one or more of: 1) direct mitigation of aerosol formation, 2) marked or complete aerosol elimination that is not dependent on human behavior, other than its inclusion in procedures, 3) low cost compared to engineering controls involving airflow (HVAC) or room construction, 4) simplicity as the irrigation solution replaces water used otherwise, 5) no additional hardware requiring sterilization between patients, 6) easily stored at room temperature, 7) widely adapted to most if not all clinical settings including those supporting poor access to care communities, 8) low tech = no training, 9) scalable from small to large organizations, 10) can be flavored/colored, and 11) does not require continuous human engagement.

In various embodiments, this water-based irrigation solution is minimally a two-part solution including water and a biocompatible high molecular weight polymer or other rheologically-similar compound of defined concentration or concentration range. A further expansion of the principles herein is for reduction of aerosol in surgical irrigation solution (typically 0.9% saline) to achieve the same reduction of aerosol in other surgical fields using water-based irrigation (for example orthopedic surgery or wound debridement). This irrigation solution is used in the ordinary dental office chairs by connection to the water line system. The irrigation solution reduces or completely eliminates aerosol generation. It is not anti-viral / bacterial nor intended to kill viruses/bacteria. Thus, clinical dentistry will still be required to use PPE to protect workers natural aerosolization (speaking, coughing, sneezing).

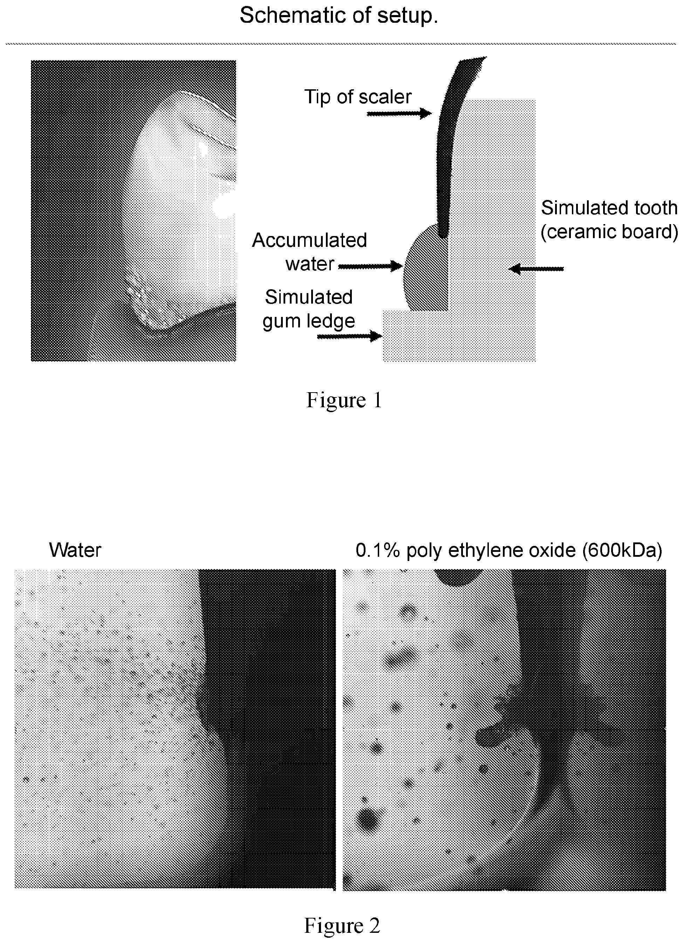

A number of embodiments can be constructed in accordance with the principles herein. Several exemplary embodiments are provided to demonstrate these principles (see Figure 1), and to demonstrate that other examples listed herein achieve results in accordance with the principles herein.

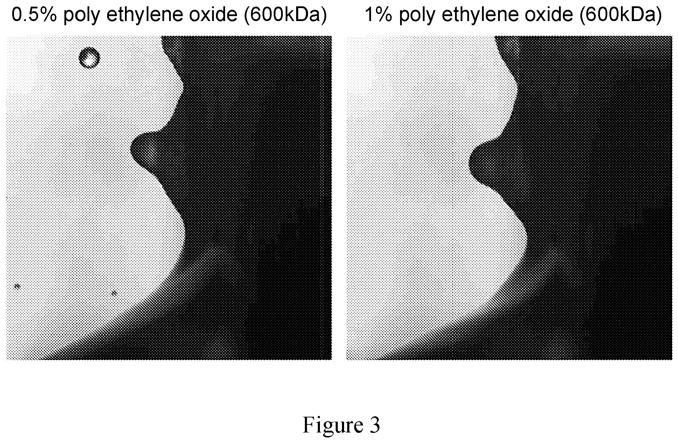

In an aspect, the present disclosure provides compositions for use in medical or dental or surgical procedures that reduce aerosolization (see Figures 2 and 3). The compositions comprise, consists essentially of, or consists of a polymer and water. In various embodiments, the compositions do not contain an organic solvent. In various embodiments, the compositions do not take the form of a personal care or cleaning product. In various embodiments, the only component other than water in a composition is a polymer. In various other embodiments, the composition further comprises one or more non-functional additives or excipients. Non-functional additives or excipients do not affect (e.g., substantially affect) the composition's shear viscosity and/or elongational viscosity, but may provide a secondary effect, such as, for example, flavoring the composition.

The compositions reduce formation of or eliminate aerosolized droplets. The term "reduction in formation of aerosolized droplets" or similar terms as used herein refers to a decrease of the number of aerosolized droplets produced during irrigation and/or mechanical agitation (such as, for example, during providing water irrigation to teeth, such as, for example, during dental procedures) of a liquid (e.g., water). For example, the amount of droplets generated during a dental cleaning procedure may be reduced. Reduction in aerosolization may result in suppression of droplet formation, including complete suppression of aerosol formation (e.g., elimination), as illustrated in Figures 2 and 3. In an embodiment, droplets having a size (diameter) of 5 $\mu$m to 100 $\mu$m, 5 $\mu$m to 75 $\mu$m, 5 $\mu$m to 50 $\mu$m, 5 $\mu$m to 25 $\mu$m, or 5 $\mu$m to 10 $\mu$m are eliminated or formation of droplets having these size ranges is reduced. The aerosolized droplets may comprise the composition, water, and/or saliva. In various other examples, the aerosolized droplets are not formed from an additional cleaning agent, such as a personal cleaning product, toothpaste, or the like, or combination thereof.

Various polymers or combinations thereof may be used in a composition of the present disclosure. Examples of polymers include, but are not limited to, poly(ethylene oxide), xanthan gum, poly(vinyl pyrrolidone), alginic acid, hyaluronic acid, chondroitin sulfate, and the like, and combinations thereof.

The aqueous polymer containing compositions of the present disclosure have desirable combinations of viscoelastic parameter. For example, a desirable viscoelasticity refers to low shear viscosity (of the order of that of water viscosity) relative to the high elongational viscosity, i.e., the ability of a liquid medium to flow under low stress (such as, for example, during passage through in liquid feed tubing) while not submitting to extensional stress (generation droplets or aerosols in the presence of rapidly rotating or oscillating surfaces or pressure differences). In an embodiment, the shear viscosity is low and the elongational viscosity is high. For example, the shear viscosity is 0.001-1000 poise, including every 0.001 poise value and range therebetween (e.g., 0.001-500, 0.001-250, 0.001-100, 0.001-50, 0.001-25, 0.001-10, 0.001-1, 0.001-0.1, 0.001-0.01, 0.01-500, 0.01-250, 0.01-100, 0.01-50, 0.01-25, 0.01-10, 0.01-1, 0.01-0.1, 0.1-500, 0.1-250, 0.1-100, 0.1-50, 0.1-25, 0.1-10, 0.1-1, 1-1000, 1-500, 1-250, 1-100, 1-50, 1-25, 1-10, 10-1000, 10-500, 10-250, 10-100, 10-50, 10-25, 100-1000, or 100-500) poise. For example, the elongational viscosity is 100-10$^7$ poise, including every integer poise value and range therebetween. In some embodiments, the elongational viscosity should be at least a magnitude or several orders of magnitude higher than the shear viscosity. For example the elongational viscosity may be 10, 100, 1000, or 10,000 or even more times higher than the shear viscosity. In another example, the elongation viscosity is 10-10,000 times larger than the shear viscosity of the aqueous composition. In various embodiments, the shear viscosity of the composition is < 6 mPas.

Viscosity may be determined by various methods known in the art. For example, intrinsic viscosity may be determined using an Ubbelohde viscometer and/or a Zimm-Crothers viscometer. For example, compositions at different concentrations of polymers may be subjected to Ubbelohde viscometry. The time of flow for a fixed volume of the composition was measured at various concentrations. The intrinsic viscosity is obtained (from the point of intersection) after extrapolation of two plots of reduced viscosity: $\eta_{red} = \eta_{sp}/C$ versus $C$ and the inherent viscosity: $\eta_{inh} = \ln(\eta_{rel})/C$ versus $C$ to zero concentration, where $C$ is the polymer concentration in g/cm$^3$. The reduced viscosity was calculated from the relation $\eta_{red} = \eta_{sp}/C = (\eta_{rel} - 1)/C$, where $\eta_{rel} = \eta/\eta_o = t/t_o$. In this expression, $\eta_o$ is the dynamic viscosity of the solvent, $t$ is the time of flow of the polymer solution, and $t_o$ is the time of flow of the solvent at the temperature of the measurement. The same procedure may be used with Zimm-Crothers viscometry.

Shear viscosity may be determined by various methods known in the art. For example, shear viscosity is determined using a rheometer. The rheometer may utilize various geometries, such as, for example, cone plate geometries and using rotational viscometers (e.g., Couette rotational viscometers). Shear viscosity may be determined at various concentrations of polymers.

Elongational viscosity may be determined by various methods known in the art. For example, elongational viscosity is measured using an elongational rheometer, which is based on the self-thinning of a liquid thread of a sample under the action of surface tension. In this device, only a single droplet of a liquid is needed to measure rheological behavior. Typically, the device comprises two plates, an upper plate and a lower plate, between which a drop of the sample is disposed. A magnetic coil is used to fix the position of the upper plate, thus forming a liquid thread of the sample. The decrease of the thread radius ($\alpha$) with time ($t$) is described by:

$$\alpha = \alpha_0 e^{-t/3\theta}$$

where $\alpha_0$ is the initial thread radius at $t = 0$, and $\theta$ is the relaxation time. The elongational viscosity ($\mu_{el}$) is in turn determined by the following equation:

$$\mu_{el} = \frac{3\theta\sigma}{2\alpha_0} e^{t/3\theta}$$

where $\sigma$ is the surface tension coefficient. Determine of elongational viscosity may also be referred to as determination viscoelastic relaxation time.

Polymers may be natural polymers and/or synthetic polymers. Examples of synthetic polymers include polyethylene oxide (which may be referred to as PEO or polyethylene glycol (PEG)), poly(vinyl pyrrolidone) (PVP). Examples of natural polymers include, but are not limited to, alginic acid, xanthan gum, hyaluronic acid, chondroitin sulfate, and the like, and combinations thereof. Additional examples include surfactants and micelles formed from said surfactants.

The polymers may have various molecular weights ($M_w$ and/or $M_n$). For example, a polymer has a molecular weight ($M_w$ or $M_n$) of 1 kDa to 10 MDa, including every 1 Da value therebetween and all ranges therebetween. For example, poly(ethylene oxide) may have a molecular weight of 1 kDa to 10 MDa, including every 1 Da value therebetween. Polyethylene oxide may have a molecular weight of 600 kDa or 8 MDa. Polyacrylic acid (PAA) may have a molecule weight of 450 kDa. Xanthan gum may have a molecular weight of 1-7 MDa. Alginic acid may have a molecular weight of 10-600 kDa. The polymers may be straight chain polymers. In some embodiments, the polymers are branched polymers.

In some embodiments, combinations of the same polymers with different molecular weights may be used (*e.g.,* a composition comprising polyethylene oxide 600 kDa and polyethylene oxide 8 MDa, each polymer having a different concentration). In various examples, combinations of the different polymers with the same or similar molecular weights ($M_w$ and/or $M_n$, wherein $M_w$ is the weight average molecular weight and $M_n$ is the number average molecular weight)) are used or combinations of different polymers with different molecular weights ($M_w$ and/or $M_n$) are used. Molecular weight of a polymer can be determined by methods known in the art, such as, for example, by comparison to polystyrene standards.

A polymer can have a range of dispersity. For example, the dispersity ($Đ$) (expressed at $M_w/M_n$) may be 1.00 to 6.00, including every 0.01 value and range therebetween. In various examples, the dispersity is 1 or greater than 1. In various embodiments, the polymers are monodisperse or polydisperse. Methods of determining the molecular weight and thus dispersity are known in the art. For example, the dispersity of the polymers is determined by comparison with polystyrene standards or by light scattering methods.

In embodiments, a polymer may have a concentration in the solution such that it is suitable for use in a dental unit waterlines. For example, the final concentration of polymer at the point of contact with the dental treatment area may be 0.01 wt% (100 ppm) to 5 wt % (50,000 ppm), including all ppm integer values and ranges therebetween, relative to the total weight of the solution. For example, the concentration can be 0.01-1 wt%, 0.01-2 wt%, 0.01-3 wt%, 0.01-4 wt%, 0.05-1 wt%, 0.05-2 wt%, 0.05-3 wt%, 0.51-4 wt%, 0.1-1 wt%, 0.1-2 wt%, 0.1-3 wt%, 0.1-4 wt%, 1-2 wt%, 1-3 wt%, or 1-4 wt%.

In various examples, a composition comprises: poly(ethylene oxide) with a molecular weight of 600 kDa having a concentration of 0.1 wt% (1000 ppm); poly(ethylene oxide) with a molecular weight of 600 kDa having a concentration of 1 wt% (10000 ppm); poly(ethylene oxide) having a molecular weight of 2 MDa at a concentration of 0.01 wt% (100 ppm); poly(ethylene oxide) having a molecular weight of 2 MDa at a concentration of 0.05 wt% (500 ppm); poly(ethylene oxide) having a molecular weight of 2 MDa at a concentration of 0.1 wt% (1000 ppm); poly(ethylene oxide) having a molecular weight of 2 MDa at a concentration of 1 wt% (10000 ppm); poly(ethylene oxide) having a molecular weight of 8 MDa at a concentration of 0.01 wt% (100 ppm); poly(ethylene oxide) having a molecular weight of 8 MDa at a concentration of 0.05 wt% (500 ppm); poly(ethylene oxide) having a molecular weight of 8 MDa at a concentration of 0.1 wt% (1000 ppm); poly(ethylene oxide) having a molecular weight of 8 MDa at a concentration of 1 wt% (10000 ppm); xanthan gum at a concentration of 0.1 wt% (1000 ppm); xanthan gum at a concentration of 0.5 wt% (5000 ppm); xanthan gum at a concentration of 1 wt% (10000 ppm); pyrrolidone having a concentration of 1 wt% (10000 ppm); pyrrolidone at a concentration of 5 wt% (50000 ppm); or alginic acid (alginate, a polysaccharide) at a concentration of 2 wt% (20000 ppm).

The polymers may be charged, uncharged, or unsubstantially uncharged. The polymers may be cationic, anionic, or zwitterionic.

A polymer suitable for the present compositions has desirable solubility in an aqueous medium (e.g., water or aqueous mixtures comprising other liquids, e.g. ethylene glycol) and remains soluble at temperatures up to the boiling point of the solvent employed. The solubility of the polymer may change at elevated temperatures relative to ambient temperature. The solubility of an agent may be from 0.001 wt.% to 25 wt.%. When added to an aqueous medium, the polymer forms a composition that has low shear viscosity and high elongational viscosity. The solubility of the polymer should be such that it is easily miscible in water.

The compositions may comprise various additives, without substantially altering the viscoelastic properties. Examples of additives include, but are not limited to, hydrogen peroxide, antioxidants or antioxidant-synergists, chelating agents, flavorings, colorings, antimicrobials, preservatives, biofilm removal agents, particles, calculus or plaque staining dyes, wetting agents/detergents, and the like, and combinations thereof.

In various embodiments, the composition further comprises one or more stabilizers. Non-limiting examples of stabilizers include those in the following table.

Table 1. Non-limiting list of stabilizers.

| Name | CAS | Stabilizer type | Concentration | Preferred concentration |
|---|---|---|---|---|
| BHT | 128-37-0 | antioxidants | 0.001-0.5% | 0.01-0.2% |
| BHA | 25013-16-5 | antioxidants | 0.001-0.5% | 0.01-0.2% |
| Ascorbic acid | 50-81-7 | antioxidants | 0.001-0.5% | 0.01-0.2% |
| Glutathion | 70-18-8 | antioxidants | 0.001-0.5% | 0.01-0.2% |
| EDTA | 6381-92-6 | Chelating agent | 0.001-0.5% | 0.01-0.2% |

Advantages of the present composition include that it does not affect downstream events, such as dental procedures, its components (such as, for example, PEO) can be washed away quickly and therefore, do not linger in the treatment area. It is may be vital for subsequently performed dental procedures that there be no detrimental effect (*e.g.,* on bond adhesion etc.) caused by the present composition.

In an aspect, this disclosure provides a dental unit system comprising a dental unit bottle and waterline configured to deliver a composition comprising an aqueous polymer composition as described herein to a dental treatment area in an individual, such that the generation of aerosol particles upon release of the composition into the dental treatment area during dental procedures is reduced (*e.g.*, relative to water alone). The system may also optionally comprise a hand-piece for controlling the release of the composition into the dental treatment area. The system may be configured to use a premade ready-to-use aerosolization reducing composition or may be configured to dilute a concentrated aerosolization reducing composition in transit at any point from the reservoir containing the concentrated composition to the point of use in the treatment area. This may be done for dental appliances with a water tank (e.g. mobile dental scaling units) by replacing the commonly used water in the tank with an embodiment of the present composition. On the other hand, non-mobile dental appliances (*e.g.*, stationary ultrasonic scalers) can be built into the dental chair and attached to the main water supply (dental unit waterline). Such appliances may have a port to continuously introduce disinfectants that prevent biofouling of the liquid feed system. This port may be used to feed the correct amount of a concentrate of present composition into the water line that is diluted to desired concentrations during its passage through the liquid feed system to the handpiece.

Some functionally relevant properties of the compositions, such as shear viscosity and flow rate in a scaling unit *(e.g.,* EMS Scaler PIEZON 250) are particularly relevant to use of the compositions. The liquid feed systems built into, for example, ultrasonic scalers employing a built-in liquid reservoir are designed for use with liquids of water-like viscosity. The rate of liquid fed to the scaler tip or other relevant parts of the instrumentation cannot be altered significantly without affecting functionality of device or requiring modification of liquid feed systems. Beneficially to fast and easy implementation, the compositions of the present disclosure may be used in such tools without modification to the tools.

In an aspect, the present disclosure may be embodied as a system having low aerosolization in a dental instrument (*e.g.*, a water syringe, air/water syringe, ultrasonic scaler, etc.) A system may have a reservoir containing one or more polymers chosen from poly(ethylene oxide), xanthan gum, poly(vinyl pyrrolidone), alginic acid, hyaluronic acid, chondroitin sulfate, and combinations thereof. A supply line is in fluid communication with the reservoir. The sully line is configured to supply the one or more plymers of the reservoir to the dental distribution as an aqueous composition at a final concentration (*i.e.,* the concentration of the aqueous composition delivered to the instrument). In some embodiments, the final concentration is such that the total concentration of the one or more polymers is 0.01 to 5 wt%, relative to the total weight of the aqueous composition. Embodiments of the system may be configured for a final concentration according to any of the compositions disclosed herein.

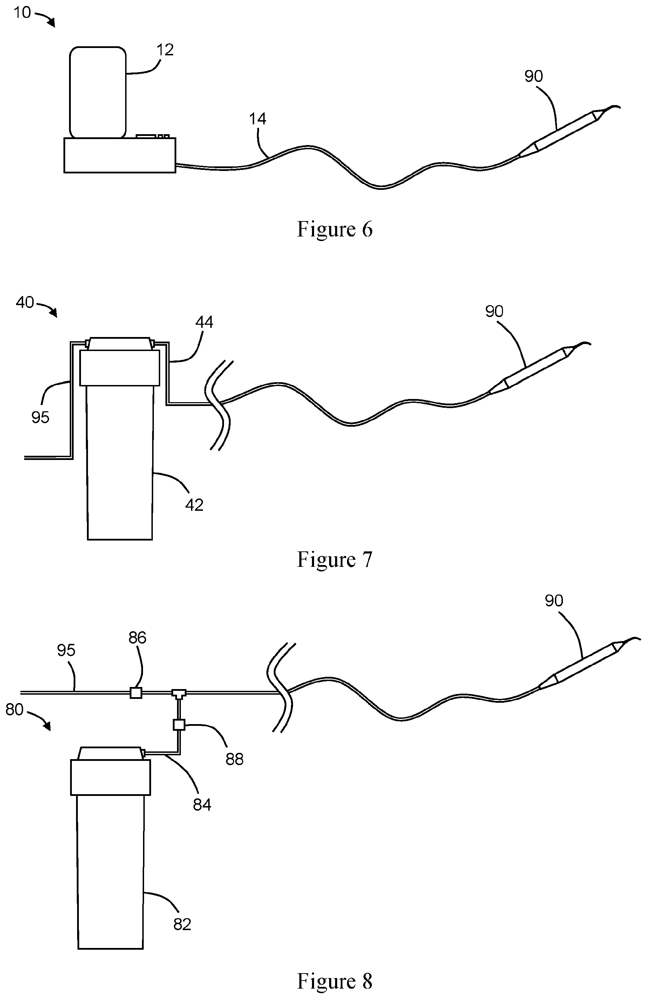

With reference to Figure 6, a system **10** for supply an aqueous composition to a dental instrument **90** may include a reservoir **12**. The reservoir **12** contains one or more polymers according to any embodiment disclosed herein. A supply line **14** is in fluid communication with the reservoir. In the system **10** depicted in Figure 6, the reservoir is a standalone (*i.e.,* independent) reservoir. As such, the one or more polymers contained within the reservoir may already be a part of an aqueous composition at the final concentration. In this way, the supply line **14** delivers the aqueous composition to the dental instrument **90** at the final concentration (the same concentration as contained within the reservoir).

With reference to Figure 7, the system **40** may be configured to attach to (*i.e.,* retrofit) an existing dental unit. For example, the reservoir **42** may be configured to be coupled in-line with the dental unit such that an inlet of the reservoir **42** is configured for connection to the dental unit waterline **95**. In this way, a flow of water from the dental unit waterline will enter the reservoir and mix with the one or more polymers contained therein. The one or more polymers contained within the reservoir may be concentrated (*e.g.*, concentrated liquid form, solid form) such that the resulting aqueous composition exiting the reservoir **42** via supply line **44** is at the desired final concentration.

In the embodiment depicted in Figure 8, a system **80** is configure to be attached to a dental unit waterline **95**. In such embodiments, the supply line **84** is configured to fluidly couple the reservoir **82** to the waterline **95** thereby introducing a material contained in the reservoir into the waterline.

In some embodiments, the system **80** further includes a flow sensor **86** configured to measure a flow of water in the dental unit waterline. The system **80** may further include an actuator **88** configured to vary a flow of the aqueous composition from the reservoir **82** based on the measured flow of water (e.g., as measured by flow sensor **86**). For example, the actuator may be a variable valve for controlling a flow of composition from the reservoir. In another example, the actuator may be a variable speed pump capable of dispensing the reservoir composition at a desired rate according to the measured flow of water in the waterline. The actuator may passively control the flow of the composition-for example, an orifice sized to control the flow at a pre-determined rate. In some embodiments, the actuator may actively control the flow of the composition-for example, an electronically- or mechanically-controlled valve that can vary the flow of the composition according to a measured flow of fluid (*e.g.*, water) through the supply line. In this way, the actuator is configured to control the flow of reservoir agent such that the characteristics of resulting aqueous composition are consistent with the values disclosed herein. For example, the actuator may be configured such that the final concentration of polymer (*e.g.*, in water, etc.) at the point of contact with the dental treatment area may be 0.01 wt% (100 ppm) to 5 wt % (50000 ppm), including all ppm integer values and ranges therebetween, relative to the total weight of the solution. For example, the concentration can be 0.01-1 wt%, 0.01-2 wt%, 0.01-3 wt%, 0.01-4 wt%, 0.05-1 wt%, 0.05-2 wt%, 0.05-3 wt%, 0.51-4 wt%, 0.1-1 wt%, 0.1-2 wt%, 0.1-3 wt%, 0.1-4 wt%, 1-2 wt%, 1-3 wt%, or 1-4 wt%. Such final concentration may be dependent on the flow of supply fluid (*e.g.,* fixed or variable), the flow of the composition (*e.g.*, fixed or variable accordingly), the concentration of polymer, etc.

In some embodiments, the polymer is added into the fluid of the supply line during flow of the fluid. For example, the compositions may be drawn into the fluid flow using the Venturi effect. In other embodiments, the reservoir may be pressurized such that the composition is injected into the supply line (*e.g.*, automatically, selectively, etc.) In such embodiments, the composition may be delivered to the supply line as a fluid.

In some embodiments, the polymer is a solid (*e.g.,* granule or other form). In such embodiments, the flow of fluid from the supply line (or a portion of the flow) may be directed into the reservoir to mix with/dissolve the polymer for subsequent (downstream) use with a patient. Such an embodiment may also be used with a fluid (*e.g.,* liquid) polymer.

In some embodiments, combinations of techniques may be used. For example, in some embodiments, a portion of flow from the supply line may be redirected to the reservoir to dissolve and/or dilute the polymer, and then subsequently re-introduced into the remaining flow of the supply line.

In an aspect, the present disclosure provides a method of reducing formation of or eliminating aerosol droplets generated during surgical irrigation procedures and dental treatment procedures. The method comprises providing an aqueous irrigation composition comprising a polymer of the present disclosure and introducing the composition into a surgical area, such as a dental treatment area, at ambient pressures or in a pressurized form, wherein the aerosolization is reduced compared to aerosolization generated by use of the aqueous composition which does not contain a polymer.

In various examples, the method reduces or eliminates aerosol particle formation during an irrigation procedure (*e.g.,* a dental procedure). In various examples, the method comprises irrigating the oral cavity of an individual with an aqueous composition comprising a polymer. The aerosolization produced from and during the dental procedure is less than the aerosolization with an aqueous medium in the absence of a polymer. In an embodiment, the composition is an irrigation composition with low aerosolization. The irrigation composition is suitable for irrigating biological cavities/surfaces or for use with dental or surgical instrumentation. The present compositions and methods may be used together with the use of ultrasonic, rotary or kinetic agitators used in treatment areas, such as dental treatment area. The present composition and methods may be used to reduce aerosolization during cleaning (*e.g.,* sterilization) of surgical or other instruments or surfaces relevant in a clinical setting, such as, for example, using an ultrasonic bath where the bath contains the composition.

The present compositions may be provided as ready-to-use or may be provided as concentrated liquids, or as powders. The concentrated liquids or powders may be prepared into usable compositions at the point of use or any point or time before that.

In various examples, the composition is supplied in various forms *(e.g.,* as concentrate or in final dilution) to the dentist or dental hygienist. Examples include where the final concentration of polyethylene oxide can be filled directly in the liquid tanks of the scaler, or a concentrate of polyethylene oxide for dilution either prior to use in scaler units with liquid tank or for automatic dilution in the feed unit of the dental chair unit (similar to hitherto employed liquid feed disinfection concentrates). Optionally, an in-can preservative can be added against microbial contamination (*e.g.*, standard FDA approved preservatives). For example, a concentrated composition has a concentration 5-100x more concentrated than 0.01 wt% (100 ppm) to 5 wt % (50,000 ppm). In embodiments, the concentrated composition may be 5-10x, 5-25x, 5-50x, 5-75x, 10-20x, 10-50x, 10-75x, 10-100x, 25-50x, 25-75x, 25-100x, 50-75x, 50-100x, or 75-100x. The concentrated composition may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100x more concentrated. For example, a concentrated composition has a concentration of 500 ppm to 99,000 ppm, including every integer ppm value and range therebetween. In embodiments, the concentrated composition has a composition of 500-1,000, 500-10,000, 500-100,000, 500-990,000, 1,000-10,000, 1,000-100,000, 1,000-990,000, 10,000-100,000, 10,000-990,000, or 100,000-990,000 ppm.

The compositions may be used in a variety of treatment procedures where irrigation liquids are used. For example, the compositions and methods may be performed on an individual who is undergoing a surgical or dental procedure. Examples of dental procedures include, but are not limited to, ultrasonic, rotary of kinetic cleaning, particle abrasion polishing, cavity preparation, extension of fissures, root canal preparation, adjustment of occlusion during restorative work, implantation, medical procedures (*e.g.*, ultrasonic debridement of wounds, water-cooled drilling procedures of bone, irrigation, and the like), events requiring air or water spray irrigation, and the like. The present compositions may be incorporated into currently existing irrigation equipment and waterlines, such as those found in a dental examination/treatment room. In various examples, the compositions may be used in an ultrasonic bath. In various other examples, the composition may be used as a coolant.

All of above aspects used in human medicine may similarly applied to veterinary use. For example, any of the above aspects may be applied to any surgical procedure procedural used in veterinary care that generates an aerosol.

In an aspect, the present disclosure provides kits. The kits may comprise components of a composition of the present disclosure or it may comprise a composition of the present disclosure dispensed into ready-to-use containers for surgical or dental procedures.

A kit may comprise separate containers for the polymer and the appropriate amount of an aqueous medium, such as water, to prepare a composition for use in rinsing a treatment area. The water or the container containing the polymer may comprise additives, or the additive many be provided separately. For example, a kit may comprise a polymer, sterile water, and optionally, additives. The polymer may be provided in any form, such as concentrated liquid or powder. The kit may further comprise disposable items, such as, for example, hoses, fittings, and other items that may be used to deliver a composition of the present disclosure. The kit may further comprise instructions for preparing and/or using the composition. The kit may further comprise one or more article for interfacing a vessel *(e.g.,* a bottle or other container containing the composition) with an irrigation system. The kit may comprise combinations of various disposable items.

A number of embodiments can be constructed in accordance with the principles herein. The following exemplary embodiments are provided to demonstrate these principles, and to demonstrate that other examples listed below achieve results in accordance with the principles herein.

## EXAMPLE 1

This example provides a description of various compositions of the present disclosure.

Solutions of various polymers at various concentrations and various molecular weights were tested using a representative scaling unit (Piezon 250, EMS (CH)). For the purpose of the test, the formulation was filled into the liquid tank of the scaling unit and the test set up described below was used to evaluate aerosol formation upon operation of the scaler.

The following compositions were tested for their ability to enhance the viscosity of dental/surgical irrigation compositions and thus reduce aerosolization, such as for use with Piezon 250.

Poly(ethylene oxide) Molecular weight 600 kDa, concentration 0.1 wt% (1000 ppm) was observed to diminish aerosolization, but did not fully suppress aerosolization.

Poly(ethylene oxide) Molecular weight 600 kDa, concentration 1 wt% (10000 ppm) received a rating of "1" as per Table 2 below.

Poly(ethylene oxide) Molecular weight 2 MDa, concentration 0.01 wt% (100 ppm) was observed to diminish aerosolization, but did not fully suppress aerosolization.

Poly(ethylene oxide) Molecular weight 2 MDa, concentration 0.05 wt% (500 ppm) nearly fully suppressed aerosolization.

Poly(ethylene oxide) Molecular weight 2 MDa, concentration 0.1 wt% (1000 ppm) fully suppressed aerosolization.

Poly(ethylene oxide) Molecular weight 2 MDa, concentration 1 wt% (10000 ppm) fully suppressed aerosolization.

Poly(ethylene oxide) Molecular weight 8 MDa, concentration 0.01 wt% (100 ppm) was observed to diminish aerosolization, but did not fully suppress aerosolization.

Poly(ethylene oxide) Molecular weight 8 MDa, concentration 0.05 wt% (500 ppm) fully suppressed aerosolization.

Poly(ethylene oxide) Molecular weight 8 MDa, concentration 0.1 wt% (1000 ppm) fully suppressed aerosolization.

Poly(ethylene oxide) Molecular weight 8 MDa, concentration 1 wt% (10000 ppm) fully suppressed aerosolization.

Xanthan gum 0.1 wt% (1000 ppm): nearly fully suppressed aerosolization (a part of some types of artificial saliva).

Xanthan gum 0.5 wt% (5000 ppm) fully suppressed aerosolization (a part of some types of artificial saliva).

Xanthan gum 1 wt% (10000 ppm) fully suppressed aerosolization (a part of some types of artificial saliva).

Polyvinylpyrrolidone 1 wt% (10000 ppm): diminished aerosolization (a part of some types of artificial saliva).

Polyvinylpyrrolidone 5 wt% (50000 ppm): significantly diminished aerosolization (a part of some types of artificial saliva).

Alginic acid (alginate, a polysaccharide) 2 wt% (20000 ppm): fully suppressed aerosolization.

Other compositions, including synthetic polymer compositions, for reducing/eliminating aerosolization produced in dental/surgical procedures comprising the above polymers can be prepared in substantially similar, suitable concentrations.

**EXAMPLE 2**

This example provides a description of quantitation of reduction of aerosolization of compositions of the present disclosure.

For the purpose of qualitative evaluation of aerosol detection, a glass slide was placed at pre-determined distances to an ultrasonic scaler tip. A simulated tooth was used to mimic the geometry of a clinical situation. Aerosol droplets generated by scaler operation were collected on the glass slide and detected qualitatively by naked eye. If no droplets were detected, the glass slide was moved closer to the scaler tip by increments, until droplets became apparent. This distance was judged to be the travel length of droplets generated by ultrasonic operation using the formulation.

Scaler flow rate was evaluated by operating the scaler for fixed periods of time and collecting total run-off.

The tests were repeated with formulations containing trace amounts of a fluorescent dye to allow quantitative evaluation of aerosol collected on the glass slides by UV/VIS spectroscopy.

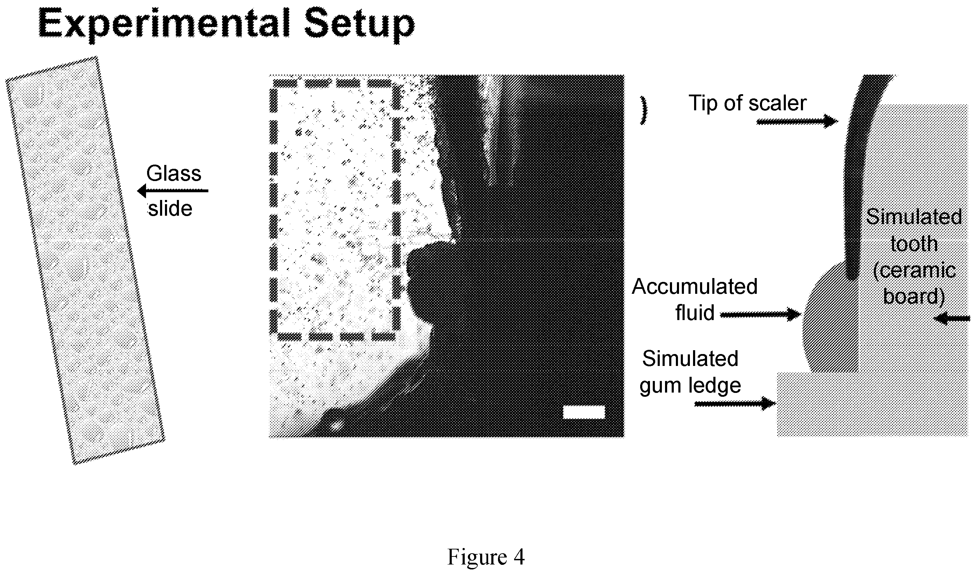

The experimental setup of the splatter test was as follows (see Figure 4). For the qualitative tests, it was determined whether there was boundary condition droplets. For quantitative tests, a sodium-fluorescein/water/polymer mixture was washed off and analyzed via UV-vis spectrophotometry, the concentration of the dye was calculated, and used to determine direct correlation of how much aerosolization/small droplets evolved.

Achieved effect of present compositions were judged on a scale of 1-4 using the following parameters.

Table 2: Rating of effect of compositions during ultrasonic scaler operation.

| Rating | Parameter |
| --- | --- |
| 1 | Aerosol or droplets detectable at >5 cm distance from scaler tip |
| 2 | Aerosol or droplets detectable at 3-5 cm distance from scaler tip |
| 3 | Aerosol or droplets detectable at 1-3 cm distance from scaler tip |
| 4 | No aerosol or droplets detectable at <1 cm distance from scaler tip |

Table 3. Qualitative Test Results:

| Polymer | Dalton | Viscosity (mPas)/shear rate at 500/s | Scaler Performance qualitative (see table 2) | Min.Distance for droplets (cm) | Scaler Flow Rate (ml/s) |
|---|---|---|---|---|---|
| **Polyethylene glycol PEG/Polyethylene oxide PEO (CAS 25322-68-3)** | 300k | 0.01%: 1.1 | 1 | >5 | 0.95 |
| | | 0.05%: 1.2 | 2 | 2 | 1.05 |
| | | 0.1%: 1.4 | 4 | <1 | 1.00 |
| | | 1.0%: 8.2 | 4 | <1 | 0.50 |
| | 600k | 0.06%: 1.3 | 2 | >1 | 0.98 |
| | | 0.1%: 1.7 | 3 | 1 | 1.00 |
| | | 0.3%: 3.1 | 4 | <1 | 0.85 |
| | | 0.6%: 6.7 | 4 | <1 | 0.65 |
| | | 0.7%: 8.2 | 4 | <1 | |
| | | 0.8%: 10.3 | 4 | <1 | |
| | | 0.9%: 12.1 | 4 | <1 | |
| | | 1.0%: 16.1 | 4 | <1 | |
| | 4000k | 0.01%: 1.1 | 1 | >5 | 0.95 |
| | | 0.05%: 1.3 | 2 | 2 | 0.95 |
| | | 0.1%: 1.4 | 4 | <1 | 0.93 |
| | | 1.0%: 48.6 | 4 | <1 | 0.15 |
| | 8000k | 0.01%: 1.2 | 4 | <1 | 0.90 |
| | | 0.1%: 25 | 4 | <1 | 0.35 |
| | | 1.0%: 68.4 | 4 | <1 | n.m. |
| | | | | | |
| **Water** | - | 1.0 | 1 | 8 | 1.05 |
| **Polyacrylic acid (PAA, CAS 9003-01-4)** | 450k | 0.1%: | 1 | >5 | 0.80 |
| | | 1.0%: 4.1 | 3 | 1 | 0.70 |
| | | 2.0%: 6.8 | 4 | <1, foam | 0.50 |
| | 4000k | 0.3%:2.5 | n.m. | n.m. | |
| | | 0.5%: 6.7 | 1 | 5, foam | 0.70 |
| | | 1.0%: 74.2 | n.m. | n.m. | |
| **Hydroxyethyl cellulose (HEC, CAS 9004-62-0)** | 370k | 0.1%: 1.6 | 1 | 10 | 0.90 |
| | | 1.0%: 28 | 3 | <1 | 0.35 |
| **Hydroxypropyl cellulose (HPC, CAS 9004-64-2)** | 370k | 0.1%: 1.6 | 1 | 10 | 1.05 |
| | | 1.0%: 28 | 3 | <1 | 0.30 |
| **Polyvinyl pyrrolidone (CAS 9003-39-8)** | 35-55k | 0.1%: 1.1 | 1 | >5 | 0.95 |
| | | 1.0%: 1.3 | 1 | >5 | 0.90 |
| | 1300k | 0.1%: 1.6 | n.m. | n.m. | |
| | | 0.5%: 2.2 | 1 | 6 | 0.90 |
| | | 1.0%: 3.3 | n.m. | n.m. | |

(continued)

| Polymer | Dalton | Viscosity (mPas)/shear rate at 500/s | Scaler Performance qualitative (see table 2) | Min.Distance for droplets (cm) | Scaler Flow Rate (ml/s) |
|---|---|---|---|---|---|
| Pluronic F-108 (CAS 9003-11-6) | 14.6k | 0.10%:1.1 | 1 | >5 | 0.90 |
| | | 1.00%:1.3 | 1 | >5 | 0.85 |
| Silwet 77-L (CAS 27306-78-1, 27252-80-8) Strongly opalescing, foaming solutions | - | 0.1%: n.m. | 1 | >5 | 0.95 |
| | | 1.0%:n.m. | 1 | >5 | 0.90 |
| Hyaluronic acid Na-salt (CAS 9067-32-7) | 50k | 0.1%:2.5 | 1 | >5 | 0.80 |
| | | 1.0%: 10.4 | 4 | <1 | 0.45 |
| | 1.5 Mio | 0.01%:2.5 | 1 | >5 | 0.75 |
| | | 0.10%: 14.7 | 4 | <1 | 0.40 |
| | | 1.0%: 215 | 1 | - | n.m. |
| Chondroitin Na-salt (CAS 39455-18-0) | | 0.1%: 1.3 | 1 | >5 | 0.85 |
| | | 1.0%: 2.3 | 1 | >5 | 0.90 |
| Mucin (CAS 84082-64-4) | brownish material, not soluble | 0.1%: n.m. | 1 | - | - |
| | | 1.0%: n.m. | | | |
| Mowiol 20-98 (Polyvinyl alcohol, CAS 9002-89-5) | 125k not soluble | 0.1%: 1.1 | 1 | - | - |
| | | 1.0%: 1.3 | 1 | - | - |
| Pullulan (CAS 9057-02-7) | | 0.1%: 1.1 | 1 | >5 | 0.85 |
| | | 1.0%: 2.0 | 1 | >5 | 0.80 |
| n.m.: not measured. | | | | | |

A range of industrial and biopolymers as well as surfactants have been tested. Polyethylene oxide (Polyethylene glycol) polymers provided desirable shear viscosities and, thus, desirable flow rates (similar to water) and elongational viscosities, leading to suppression of droplet or aerosol formation. Polysaccharides like hydroxyethyl or hydroxypropyl celluloses also provided desirable properties, but in higher concentrations (1%) and at higher shear viscosities, consequentially inducing lower flow rates. Hyaluronic acid also provided desirable performance at 0.1% but with lower flow rate.

Table 4. Quantitative tests via splatter and evaluation with fluorescent dye (Na-fluorescein):

| [Fluorescein] % | [PEG] % | PEG | [Fluorescein] ppm | normed [Fluorescein] ppm | distance to glass slide [cm] | measured [Fluorescein] [ppm] | relative [Fluorescein] measured [ppm] | lg(relative [Fluorescein] measured/distance^2) [lg(ppm/cm^2)] | Average [lg(ppm/cm^2)] | Shear Viscosity [mPa*s] | Flow Rate [ml/s] |
|---|---|---|---|---|---|---|---|---|---|---|---|
| 0.1044 | 0.9990 | PEG 300 kDa | 1044 | 1000 | 1.00 | 0.063 | 6.30E-05 | -4.201 | -4.294 | n.m. | 0.5 |
| 0.1044 | 0.9990 | PEG 300 kDa | 1044 | 1000 | 1.00 | 0.041 | 4.10E-05 | -4.387 | | | |
| 0.1005 | 0.0999 | PEG 300 kDa | 1005 | 1000 | 1.00 | 0.333 | 3.33E-04 | -3.478 | -3.489 | 1.33 | 0.9 |
| 0.1005 | 0.0999 | PEG 300 kDa | 1005 | 1000 | 2.00 | 0.079 | 7.90E-05 | -3.500 | | | |
| 0.0994 | 0.0746 | PEG 300 kDa | 994 | 990 | 3.00 | 0.133 | 1.34E-04 | -2.917 | -2.580 | 1.26 | 0.9 |
| 0.0994 | 0.0746 | PEG 300 kDa | 994 | 990 | 5.00 | 0.226 | 2.28E-04 | -2.243 | | | |
| 0.0993 | 0.0500 | PEG 300 kDa | 993 | 988 | 9.00 | 0.062 | 6.28E-05 | -2.294 | -2.384 | 1.17 | 0.95 |
| 0.0993 | 0.0500 | PEG 300 kDa | 993 | 988 | 9.00 | 0.041 | 4.15E-05 | -2.473 | | | |
| 0.1013 | 0.0250 | PEG 300 kDa | 1013 | 1008 | 10.00 | 0.054 | 5.36E-05 | -2.271 | -2.241 | 1.11 | 0.95 |
| 0.1013 | 0.0250 | PEG 300 kDa | 1013 | 1008 | 10.00 | 0.062 | 6.15E-05 | -2.211 | | | |

(continued)

| [Fluorescein] % | [PEG] % | PEG | [Fluorescein] ppm | normed [Fluorescein] ppm | distance to glass slide [cm] | measured [Fluorescein] [ppm] | relative [Fluorescein] messured [ppm] | lg(relative [Fluorescein] messured/distance^2) [lg(ppm/cm^2)] | Average [lg(ppm/cm^2)] | Shear Viscosity [mPa*s] | Flow Rate [ml/s] |
|---|---|---|---|---|---|---|---|---|---|---|---|
| 0.1026 | 1.0338 | PEG 600 kDa | 1026 | 1021 | 1.00 | 0.043 | 4.21E-05 | -4.375 | -4.375 | n.m | 0.35 |
| 0.1026 | 1.0338 | PEG 600 kDa | 1026 | 1021 | 1.00 | 0.373 | 3.65E-04 | -3.437 | | | |
| 0.1080 | 0.4876 | PEG 600 kDa | 1080 | 1075 | 1.00 | 0.038 | 3.53E-05 | -4.452 | -4.489 | 5.04 | 0.6 |
| 0.1080 | 0.4876 | PEG 600 kDa | 1080 | 1075 | 1.00 | 0.032 | 2.98E-05 | -4.526 | | | |
| 0.1157 | 0.2440 | PEG 600 kDa | 1157 | 1151 | 1.00 | 0.044 | 3.82E-05 | -4.418 | -4.549 | 2.53 | 0.8 |
| 0.1157 | 0.2440 | PEG 600 kDa | 1157 | 1151 | 1.00 | 0.024 | 2.08E-05 | -4.681 | | | |
| 0.1135 | 0.1219 | PEG 600 kDa | 1135 | 1129 | 2.00 | 0.020 | 1.77E-05 | -4.150 | -4.150 | 1.66 | 0.9 |
| 0.1135 | 0.1219 | PEG 600 kDa | 1135 | 1129 | 2.00 | 0.311 | 2.75E-04 | -2.958 | | | |
| 0.1246 | 0.0610 | PEG 600 kDa | 1246 | 1240 | 5.00 | 0.073 | 5.89E-05 | -2.832 | -2.963 | 1.3 | 0.95 |
| 0.1246 | 0.0610 | PEG 600 kDa | 1246 | 1240 | 5.00 | 0.040 | 3.23E-05 | -3.093 | | | |

| [Fluorescein] % | [PEG] % | PEG | [Fluorescein] ppm | normed [Fluorescein] ppm | distance to glass slide [cm] | measured [Fluorescein] [ppm] | relative [Fluorescein] messured [ppm] | lg(relative [Fluorescein] messured/distance^2) [lg(ppm/cm^2)] | Average [lg(ppm/cm^2)] | Shear Viscosity [mPa*s] | Flow Rate [ml/s] |
|---|---|---|---|---|---|---|---|---|---|---|---|
| 0.1068 | 0.1101 | PEG 8 Mio | 1068 | 1063 | 1.00 | 0.000 | 0.00E+00 | | -5.549 | 3.75 | 0.4 |
| 0.1068 | 0.1101 | PEG 8 Mio | 1068 | 1063 | 1.00 | 0.003 | 2.82E-06 | -5.549 | | | |
| 0.1087 | 0.0552 | PEG 8 Mio | 1087 | 1082 | 1.00 | 0.002 | 1.85E-06 | -5.733 | -5.645 | 2.1 | 0.6 |
| 0.1087 | 0.0552 | PEG 8 Mio | 1087 | 1082 | 1.00 | 0.003 | 2.77E-06 | -5.557 | | | |
| 0.0985 | 0.0282 | PEG 8 Mio | 985 | 980 | 1.00 | 0.008 | 8.16E-06 | -5.088 | -4.801 | 1.53 | 0.75 |
| 0.0985 | 0.0282 | PEG 8 Mio | 985 | 980 | 1.00 | 0.030 | 3.06E-05 | -4.514 | | | |
| 0.0993 | 0.0136 | PEG 8 Mio | 993 | 988 | 2.00 | 0.050 | 5.06E-05 | -3.694 | -3.143 | 1.22 | 0.85 |
| 0.0993 | 0.0136 | PEG 8 Mio | 993 | 988 | 5.00 | 0.101 | 1.02E-04 | -2.592 | | | |
| 0.0967 | 0.0078 | PEG 8 Mio | 967 | 962 | 5.00 | 0.029 | 3.01E-05 | -3.123 | -3.048 | 1.11 | 0.8 |
| 0.0967 | 0.0078 | PEG 8 Mio | 967 | 962 | 5.00 | 0.041 | 4.26E-05 | -2.973 | | | |
| 0.0962 | 0.0038 | PEG 8 Mio | 962 | 957 | 7.00 | 0.031 | 3.24E-05 | -2.799 | -2.461 | 1.12 | 0.9 |
| 0.0962 | 0.0038 | PEG 8 Mio | 962 | 957 | 7.00 | 0.147 | 1.54E-04 | -2.123 | | | |
| 0.0987 | 0.0019 | PEG 8 Mio | 987 | 983 | 14.00 | 0.051 | 5.19E-05 | -1.993 | -2.196 | 1 | 0.9 |
| 0.0987 | 0.0019 | PEG 8 Mio | 987 | 983 | 14.00 | 0.020 | 2.04E-05 | -2.399 | | | |

n.m.: not measured.

The distance between the scaler and the glass slide was set to the minimum distance-*i.e.*, where droplets were observed on the slide (see Figure 4). A constant was introduced by the logarithmic concentration of fluorescein divided by the distance of the scaler to the glass slide. By multiplying it with the specific flow rate for the PIEZON 250 scaler, a weighted performance can be attributed (see Table 4). Additionally, simulated clinical evaluations were performed in a qualitative manner using a Dentsply Cavitron scaler. Some experiments were conducted at a water supply pressure of 26.5 psi and a flow rate of 40.6 ml/min (based on the manufacturer's guideline for the Cavitron, the water supply must be 25 psi (172 kPa) minimum to 60 psi (414 kPa) maximum).

Best performance was seen with Polyethylene glycol 600 kDa and concentrations of 1200-2400 ppm, Polyethylene glycol 8 MDa 280 ppm.

It goes without saying, that a reduction in droplet or aerosol formation positively influences operators' ability to view the field of operation. This effect is especially prominent and helpful when using magnifying loupes and even more so when illuminating the field of operation using high intensity lights.

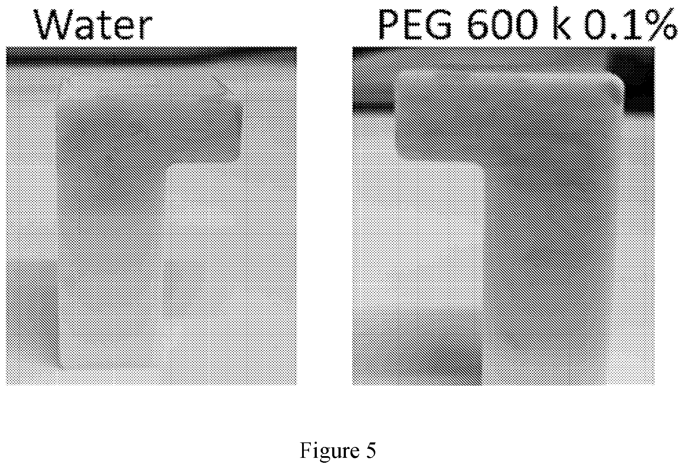

Cleaning effectiveness: To verify that introduction of a polymer does not negatively influence cleaning capacity of dental ultrasonic scalers, the cleaning effectiveness in a model system was studied. For this, the fluoride varnish Fluorprotector S was dyed with a perylene dye (Fluorescent Red) (see Figure 5) and the to be scaled zirconia model was coated with the adjusted material. The coated and dried block was cleaned with the PIEZON hand piece either with water or the formulated polyethylene oxide/water solution. In both trials the varnish was removed quickly with the scaler unit in a timeframe of 30 seconds. This proves that use of the composition does not entail a reduction of cleaning efficacy in dental procedures.

Although the present disclosure has been described with respect to one or more particular embodiments and/or examples, it will be understood that other embodiments and/or examples of the present disclosure may be made without departing from the scope of the present disclosure.

**Claims**

1. A system (40; 80) for irrigation of a treatment area, comprising:

   a reservoir (42; 82) containing one or more polymers chosen from poly(ethylene oxide), xanthan gum, poly(vinyl pyrrolidone), alginic acid, hyaluronic acid, chondroitin sulfate, and combinations thereof;
   a dental instrument (90); and
   a supply line (44; 84) in fluid communication with the reservoir (42; 82), the supply line (44; 84) configured to supply the one or more polymers of the reservoir (42; 82) to the dental instrument (90) as an aqueous composition, wherein the reservoir (42; 82) has an inlet configured to connect to a dental unit waterline (95), **characterised in that** the dental instrument (90) is an ultrasonic scaler that is in fluid communication with the supply line (44; 84) and is supplied with a flow of the aqueous composition at a final total concentration of the one or more polymers of 0.01 wt% to 5 wt% relative to the total weight of the composition and wherein the composition exhibits a shear viscosity of $10^{-3}$ to $10^3$ poise and an elongational viscosity of $10^2$ to $10^7$ poise.

2. The system (40; 80) of claim 1, wherein the one or more polymers of the reservoir (42; 82) are in an aqueous composition at a first concentration, and the supply line (44; 84) is configured to connect to a dental unit waterline (95) to provide the aqueous composition to the dental unit waterline (95).

3. The system (80) of claim 2, further comprising a flow sensor (86) configured to measure a flow of water in the dental unit waterline (95) and/or wherein the supply line (84) further comprises an actuator (88) configured to vary a flow of the aqueous composition from the reservoir (82) based on the measured flow of water in the dental unit waterline (95).

4. The system (40; 80) according to any one of claims 1 to 3, wherein the elongational viscosity of the composition is 10-10,000 times larger than the shear viscosity of the composition.

5. The system (40; 80) according to any one of claims 1-4, wherein the aqueous composition further comprises one or more additives.

6. The system (40; 80) according to claim 5, wherein the one or more additives are chosen from hydrogen peroxide, antioxidants or antioxidant-synergists, chelating agents, flavorings, colorings, antimicrobials, preservatives, biofilm removal agents, particles, calculus or plaque staining dyes, wetting agents/detergents, and combinations thereof.

**Patentansprüche**

1. System (40; 80) zur Spraybenetzung eines Behandlungsbereichs, umfassend:

 einen Vorratsbehälter (42; 82), der ein oder mehrere Polymere enthält, ausgewählt aus Poly(ethylenoxid), Xanthangummi, Poly(vinylpyrrolidon), Alginsäure, Hyaluronsäure, Chondroitinsulfat und Kombinationen davon; ein zahnärztliches Instrument (90); und eine Zufuhrleitung (44; 84), die in Durchflussverbindung mit dem Vorratsbehälter (42; 82) steht, wobei die Zufuhrleitung (44; 84) so konfiguriert ist, dass sie dem zahnärztlichen Instrument (90) das eine oder die mehreren Polymere aus dem Vorratsbehälter (42; 82) als wässrige Zusammensetzung zuführt, wobei der Vorratsbehälter (42; 82) einen Einlass aufweist, der so konfiguriert ist, dass er mit einer Wasserleitung (95) einer Dentaleinheit verbunden werden kann, **dadurch gekennzeichnet, dass** das zahnärztliche Instrument (90) ein Ultraschall-Scaler ist, der in Durchflussverbindung mit der Zufuhrleitung (44; 84) steht und mit einem Strom der wässrigen Zusammensetzung mit einer finalen Gesamtkonzentration von 0,01 Gew.-% bis 5 Gew.-% des einen oder der mehreren Polymere, bezogen auf das Gesamtgewicht der Zusammensetzung, versorgt wird, und wobei die Zusammensetzung eine Scherviskosität von $10^{-3}$ bis $10^3$ Poise und eine Dehnviskosität von $10^2$ bis $10^7$ Poise aufweist.

2. System (40; 80) nach Anspruch 1, wobei das eine oder die mehreren Polymere des Vorratsbehälters (42; 82) in einer wässrigen Zusammensetzung in einer ersten Konzentration vorliegen und die Zufuhrleitung (44; 84) so konfiguriert ist, dass sie mit einer Wasserleitung (95) der Dentaleinheit verbunden werden kann, um die wässrige Zusammensetzung der Wasserleitung (95) der Dentaleinheit zuzuführen.

3. System (80) nach Anspruch 2, das weiterhin einen Durchflusssensor (86) umfasst, der so konfiguriert ist, dass er einen Wasserdurchfluss in der Wasserleitung (95) der Dentaleinheit misst, und/oder wobei die Zufuhrleitung (84) weiterhin einen Aktuator (88) umfasst, der so konfiguriert ist, dass er den Strom der wässrigen Zusammensetzung aus dem Vorratsbehälter (82) auf der Grundlage des gemessenen Wasserdurchflusses in der Wasserleitung (95) der Dentaleinheit variiert.

4. System (40; 80) nach einem der Ansprüche 1 bis 3, wobei die Dehnviskosität der Zusammensetzung 10- bis 10.000-mal größer ist als die Scherviskosität der Zusammensetzung.

5. System (40; 80) nach einem der Ansprüche 1 bis 4, wobei die wässrige Zusammensetzung weiterhin ein oder mehrere Additive enthält.

6. System (40; 80) nach Anspruch 5, wobei das eine oder die mehreren Additive ausgewählt sind aus Wasserstoffperoxid, Antioxidantien oder Antioxidans-Synergisten, Chelatbildnern, Aromastoffen, Farbstoffen, antimikrobiellen Mitteln, Konservierungsmitteln, Mitteln zur Entfernung von Biofilmen, Partikeln, Farbstoffen zum Anfärben von Zahnstein oder Plaque, Netzmitteln/Reinigungsmitteln und Kombinationen davon.

**Revendications**

1. Système (40 ; 80) d'irrigation pour une zone de traitement, comprenant :

 un réservoir (42 ; 82) contenant un ou plusieurs polymères choisis parmi le poly(oxyde d'éthylène), la gomme de xanthane, la polyvinylpyrrolidone, l'acide alginique, l'acide hyaluronique, le sulfate de chondroïtine, et leurs combinaisons ; un instrument dentaire (90) ; et une conduite d'alimentation (44 ; 84) en communication fluide avec le réservoir (42 ; 82), la conduite d'alimentation (44 ; 84) étant configurée pour fournir lesdits un ou plusieurs polymères du réservoir (42 ; 82) à l'instrument dentaire (90) sous forme d'une composition aqueuse, dans lequel le réservoir (42 ; 82) a une entrée configurée pour se connecter à une conduite d'eau d'équipement dentaire (95), **caractérisé en ce que** l'instrument dentaire (90) est un détartreur à ultrasons qui est en communication fluide avec la conduite d'alimentation (44 ; 84) et qui est alimenté avec un flux de la composition aqueuse ayant une concentration totale finale desdits un ou plusieurs polymères de 0,01 % en poids à 5 % en poids relativement au poids total de la composition et dans lequel la composition présente une viscosité de cisaillement de $10^{-3}$ à $10^3$ poise et une viscosité élongationnelle de $10^2$ à $10^7$ poise.

**2.** Système (40 ; 80) selon la revendication 1, dans lequel lesdits un ou plusieurs polymères du réservoir (42 ; 82) sont sous la forme d'une composition aqueuse ayant une première concentration, et la conduite d'alimentation (44 ; 84) est configurée pour se connecter à une conduite d'eau d'équipement dentaire (95) pour fournir la composition aqueuse à la conduite d'eau d'équipement dentaire (95).

**3.** Système (80) selon la revendication 2, comprenant en outre un capteur de débit (86) configuré pour mesurer un débit d'eau dans la conduite d'eau d'équipement dentaire (95) et/ou dans lequel la conduite d'alimentation (84) comprend en outre un actionneur (88) configuré pour faire varier un débit de la composition aqueuse provenant du réservoir (82) en fonction du débit d'eau mesuré dans la conduite d'eau d'équipement dentaire (95).

**4.** Système (40 ; 80) selon l'une quelconque des revendications 1 à 3, dans lequel la viscosité élongationnelle de la composition est de 10 à 10 000 fois plus grande que la viscosité de cisaillement de la composition.

**5.** Système (40 ; 80) selon l'une quelconque des revendications 1 à 4, dans lequel la composition aqueuse comprend en outre un ou plusieurs additifs.

**6.** Système (40 ; 80) selon la revendication 5, dans lequel lesdits un ou plusieurs additifs sont choisis parmi le peroxyde d'hydrogène, les antioxydants ou les synergistes antioxydants, les agents chélateurs, les aromatisants, les colorants, les antimicrobiens, les agents de conservation, les agents d'élimination de biofilm, les particules, les agents de coloration du tartre ou de la plaque, les agents mouillants/détergents, et leurs combinaisons.

## Schematic of setup.

Tip of scaler

Simulated tooth
(ceramic board)

Accumulated
water

Simulated
gum ledge

Figure 1

Water

0.1% poly ethylene oxide (600kDa)

Figure 2

0.5% poly ethylene oxide (600kDa)    1% poly ethylene oxide (600kDa)

Figure 3

# Experimental Setup

Figure 4

EP 4 157 138 B1

Figure 5

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

*   US 2006254988 A1

### Non-patent literature cited in the description

*   *CHEMICAL ABSTRACTS*, 128-37-0
*   *CHEMICAL ABSTRACTS*, 25013-16-5
*   *CHEMICAL ABSTRACTS*, 50-81-7
*   *CHEMICAL ABSTRACTS*, 70-18-8
*   *CHEMICAL ABSTRACTS*, 6381-92-6
*   *CHEMICAL ABSTRACTS*, 27306-78-1
*   *CHEMICAL ABSTRACTS*, 27252-80-8
*   *CHEMICAL ABSTRACTS*, 9067-32-7
*   *CHEMICAL ABSTRACTS*, 39455-18-0
*   *CHEMICAL ABSTRACTS*, 84082-64-4
*   *CHEMICAL ABSTRACTS*, 9002-89-5
*   *CHEMICAL ABSTRACTS*, 9057-02-7